# EUROPÄISCHE PATENTANMELDUNG

(11) **EP 0 560 150 A1**
(43) Veröffentlichungstag der Anmeldung: **15.09.1993**
(21) Anmeldenummer: 93103168.6
(22) Anmeldetag: 27.02.1993
(51) Int. Cl.: G01N 15/08, G01N 33/24, E02D 1/02

(54) **Verfahren und Vorrichtung zur Bestimmung der geohydraulischen Durchlässigkeit von grundwasserdurchströmten Bodenbereichen**

(30) Priorität: 11.03.1992 DE 4207692
(71) Anmelder: IEG Industrie-Engineering GmbH, D-72770 Reutlingen (DE)
(72) Erfinder: Bernhardt, Bruno, W-7410 Reutlingen (DE)
(74) Vertreter: Möbus, Rudolf, Dipl.-Ing.

(57) **Zusammenfassung**

Verfahren und Vorrichtung zur Messung der geohydraulischen Durchlässigkeit von Bodenbereichen (11) durch Erzeugen von Unterdruck in einem Brunnenschacht (10) und Messung des dadurch bewirkten Anstiegs (27) bzw. des Wiederabsinkens des Grundwasserspiegels (24) im Brunnenschacht unter Vermeidung eines Abpumpens von Grundwasser aus dem Schacht (10).

## Beschreibung

Die Erfindung betrifft ein Verfahren und eine Vorrichtung zur Bestimmung der geohydraulischen Durchlässigkeit von grundwasserdurchströmten Bodenbereichen, insbesondere für die Dimensionierung eines einen Grundwasser-Kreislauf erzeugenden Brunnens.

Die Bestimmung der geohydraulischen Durchlässigkeit von Bodenbereichen ist wichtig für die Dimensionierung von Brunnen, die in diese Bodenbereiche eingebracht werden sollen. Nach dem Grad der Durchlässigkeit des Bodenbereichs bestimmt sich beispielsweise die Tiefe und der Durchmesser des Brunnenschachts sowie die Auswahl der Filter, mit denen der Brunnenschacht ausgekleidet wird. Besondere Bedeutung haben die Durchlässigkeitsbestimmungen dabei für Brunnen, die einen Grundwasser-Kreislauf im den Brunnenschacht umgebenden Erdreich erzeugen. Die Reichweite dieses Grundwasser-Kreislaufs hängt stark von der geohydraulischen Durchlässigkeit des Bodenbereichs sowie deren Anisotropie, d.h. deren unterschiedliche Größe in verschiedenen Richtungen, ab. Insbesondere das Verhältnis der Durchlässigkeit in horizontaler zu der in vertikaler Strömungsrichtung ist hierbei von Bedeutung. Solche einen Grundwasser-Kreislauf erzeugenden Brunnen werden insbesondere zur Entfernung von Kontaminationen in von Grundwasser durchströmtem Erdreich eingesetzt. Nur durch die Bestimmung der geohydraulischen Durchlässigkiet des kontaminierten Erdreichs läßt sich vorausberechnen, wie der Brunnen dimensioniert werden muß, um den gesamten kontaminierten Bodenbereich erfassen zu können, und ob gegebenenfalls ein zweiter Brunnen notwendig ist.

Bislang wird die geohydraulische Durchlässigkeit von Böden durch Pumpversuche bestimmt. Dazu werden Brunnen in den zu untersuchenden Bodenbereich gebohrt, aus denen Grundwasser abgepumpt wird. Durch das Abpumpen senkt sich der Grundwasserspiegel in der Umgebung des Brunnenschachtes ab. Der Grad der Absenkung des Grundwasserspiegels wird durch Peilrohre, die in unterschiedlicher Entfernung zum Brunnenschacht in den Boden eingebracht werden, bestimmt. Aus der dem Brunnenschacht entnommenen Wassermenge und dem Grad der Absenkung des Grundwasserspiegels in der Umgebung des Brunnenschachtes kann dann auf die Durchlässigkeit des Bodens geschlossen werden. Diese bekannten Verfahren haben den Nachteil, daß sie in bebauten Gegenden nicht durchgeführt werden können, da durch die Absenkung des Grundwasserspiegels auch ein Absenken von Gebäuden in der Nähe des Brunnenschachtes möglich ist. Außerdem sind sie durch die Notwendigkeit, Peilrohre in der Umgebung des Brunnenschachtes zur Messung der Absenkung des Grundwasserspiegels einbringen zu müssen, relativ aufwendig und benötigen ein großes Meßgebiet. Ein weiterer Nachteil besteht darin, daß das dem Brunnenschacht entnommene Grundwasser wieder ins Erdreich zurückgeführt werden muß. Dies muß in einiger Entfernung vom Brunnenschacht geschehen, um eine Verfälschung der Meßergebnisse zu vermeiden, was jedoch sehr aufwendig ist.

Der Erfindung liegt die Aufgabe zugrunde, ein Verfahren und eine Vorrichtung zur Bestimmung der geohydraulischen Durchlässigkeit ohne Abpumpen von Grundwasser unter Vermeidung der obengenannten Nachteile zu schaffen.

Die Aufgabe wird mit einem Verfahren der eingangs genannten Art erfindungsgemäß dadurch gelöst, daß in einem Brunnenschacht im zu untersuchenden Bodenbereich oberhalb des Grundwasserspiegels ein Unterdruck einstellbarer Größe erzeugt wird und der dadurch bewirkte Anstieg des Grundwasserspiegels im Brunnenschacht pro Zeiteinheit sowie der dort herrschende Unterdruck und/oder das Absinken des Grundwasserspiegels nach Beseitigung des Unterdrucks gemessen und aus diesen Meßwerten die geohydraulische Durchlässigkeit des untersuchten Bodenbereichs berechnet wird.

Im Gegensatz zu den Pumpversuchen tritt hier keine Absenkung, sondern im Gegenteil ein Anstieg des Grundwasserspiegels in der Umgebung des Brunnenschachtes auf. Das Verfahren ist somit auch in bebauten Gegenden einsetzbar. Da dem Brunnenschacht zudem kein Grundwasser entnommen wird, entfällt auch das Problem der Wiederzuleitung von Grundwasser ins Erdreich. Es sind auch keine Peilrohre in der Umgebung des Brunnenschachts zur Messung der Veränderung des Grundwasserspiegels erforderlich. Die Messung der Veränderung des Grundwasserspiegels wird allein im Brunnenschacht selbst vorgenommen. Aus dem gemessenen Anstieg bzw. Absinken des Grundwasserspiegels pro Zeiteinheit und dem im Brunnenschacht herrschenden Unterdruck kann die geohydraulische Durchlässigkeit des Bodens mindestens ebenso zuverlässig bestimmt werden wie bei den herkömmlichen Pumpversuchen. Das neue Verfahren stellt somit eine preiswerte, weil konstruktiv einfach zu verwirklichende, zuverlässige und überall einsetzbare Alternative zu den bislang eingesetzten Messungen durch Pumpversuche dar.

Die Messungen können auch nacheinander bei Zufluß von Grundwasser aus verschiedenen Richtungen in den Brunnenschacht durchgeführt und daraus die Anisotropie der geohydraulischen Durchlässigkeit des Bodenbereichs berechnet werden. Insbesondere kann zunächst der Anstieg oder das Absinken des Grundwasserspiegels bei Zufluß von Grundwasser aus horizontaler Richtung und anschließend aus vertikaler Richtung in dem Brunnenschacht gemessen werden. Entsprechend der festgestellten Anisotropie kann dann die Dimensionierung eines in das untersuchte Erdreich einzubringenden Brunnens vorgenommen werden. Es kann auch die horizontale Durchlässigkeit des Bodenbereichs in unterschiedlichen Tiefen gemessen werden. Dies ist insbesondere für die Dimensionierung von Brunnen mit einem Grundwasser-Kreislauf notwendig, bei denen der Brunnenschacht in zwei horizontale Bereiche aufgeteilt ist. Aus den Durchlässigkeitsmessungen in unterschiedlicher Tiefe läßt sich die notwendige Schachttiefe und der günstigste Ort für eine Trennwandung zur Abteilung der beiden Schachtbereiche bestimmen. Wird zusätzlich die vertikale Durchlässigkeit des Bodenbereichs gemessen, läßt sich auch die Reichweite des Brunnens bestimmen.

Die Vorrichtung zur Durchführung des Verfahrens weist einen Brunnenschacht mit bereichsweise wasserdurchlässiger Schachtwandung, wobei der Brunnenschacht oberhalb des Grundwasserspiegels luft- und wasserundurchlässig abgedichtet ist, Einrichtungen zur Unterdruckerzeugung und eine Meßsonde zur Messung der Veränderung des Grundwasserspiegels pro Zeiteinheit und des im Schacht herrschenden Unterdrucks auf. Die Einrichtung zur Unterdruckerzeugung kann dabei aus einer Saugpumpe, einer Vakuumkammer und einem Ventil bestehen und außerhalb des Brunnenschachts angeordnet und mit diesem über ein Rohr verbunden sein. Mit der Saugpumpe wird zunächst in der Vakuumkammer ein bestimmter Unterdruck erzeugt, der durch Öffnen eines Ventils auf den Brunnenschacht übertragen wird. Auf diese Weise ist der im Schacht herrschende Unterdruck bequem von außen auf den gewünschten Wert einstellbar. Die Meßsonde kann mit einer außerhalb des Brunnenschachts angeordneten Auswerteeinrichtung verbunden sein, um die aufgenommenen Meßwerte auslesen oder auch weiterverarbeiten zu können.

Zur Messung der horizontalen geohydraulischen Durchlässigkeit des zu untersuchenden Bodenbereichs kann der Brunnenschacht vorteilhafterweise einen oder mehrere horizontale Bereiche mit wasserdurchlässiger Schachtwandung, die gegeneinander abgedichtet sind, aufweisen. Bei mehreren horizontalen Schachtbereichen können der oder die oberen Schachtbereiche durch ein durch die Abdichtungen zwischen den Bereichen bis oberhalb des Grundwasserspiegels im Brunnenschacht geführtes Rohr überbrückbar sein. Auf diese Weise können nacheinander Durchlässigkeitsmessungen bei Zufluß von Grundwasser in verschiedenen Tiefen in den Brunnenschacht vorgenommen werden. Dadurch können evtl. vorhandene Schichten unterschiedlicher Wasserdurchlässigkeit im Boden ermittelt werden. Zur Messung der vertikalen geohydraulischen Durchlässigkeit des zu untersuchenden Bodenbereichs kann der Brunnenschacht von einem ins Erdreich getriebenen, unten offenen Rohr gebildet sein, dessen Inneres nur im oberhalb des Grundwasserspiegels liegenden Bereich von Erdreich befreit ist. Auf diese Weise wird Grundwasser ausschließlich durch die untere Rohröffnung in den mit Erdreich angefüllten Brunnenschacht eingesaugt und muß das dort befindliche Erdreich in vertikaler Richtung durchströmen. Ein horizontaler Zufluß von Grundwasser ist unmöglich, die Messung der vertikalen Durchlässigkeit des Bodens damit sehr genau. Das Rohr kann dabei einen nur kleinen Durchmesser aufweisen und in den zu untersuchenden Bodenbereich einschlagbar oder eindrehbar sein. Eine andere Möglichkeit zur Messung der vertikalen Durchlässigkeit stellt die Ausbildung des Brunnenschachts aus drei konzentrischen Rohren dar, wobei das mittlere Rohr an seinem unteren Ende einen Drehmeißel aufweist und an seinem oberen Ende mit einem Drehantrieb verbunden ist und das innere Rohr zusammen mit dem Bohrkern aus dem mittleren Rohr herausnehmbar ist. Die drei Rohre werden mit Hilfe des mittleren Rohres bis zum Grundwasserspiegel in das Erdreich eingebohrt. Anschließend wird das innere Rohr mit dem in ihm enthaltenen Bohrkern aus dem mittleren Rohr entnommen, vom Bohrkern befreit und wieder ins mittlere Rohr eingesetzt. Danach wird die gesamte Anordnung weiter ins Erdreich bis zur gewünschten Tiefe eingebohrt, wobei das innere und das äußere Rohr jeweils als Stützrohre für das mittlere Rohr dienen, das die Anordnung über den Drehantrieb in den Boden treibt. Zur Erleichterung des Bohrvorgangs kann eine Einrichtung zum Einpressen von Spülwasser in den Raum zwischen den Rohren zum Ausschwemmen von beim Bohrvorgang gelöstem Erdreich vorgesehen sein.

Nachfolgend werden bevorzugte Ausführungsbeispiele einer erfindungsgemäßen Vorrichtung anhand der Zeichnung näher erläutert.

Im einzelnen zeigen:
- Fig. 1: einen Längsschnitt durch eine erste Ausführungsform der Vorrichtung;
- Fig. 2: einen Längsschnitt durch eine zweite Ausführungsform einer Vorrichtung;
- Fig. 3: einen Längsschnitt durch eine dritte Ausführungsform der Vorrichtung;
- Fig. 4: einen Längsschnitt durch eine vierte Ausführungsform der Vorrichtung.

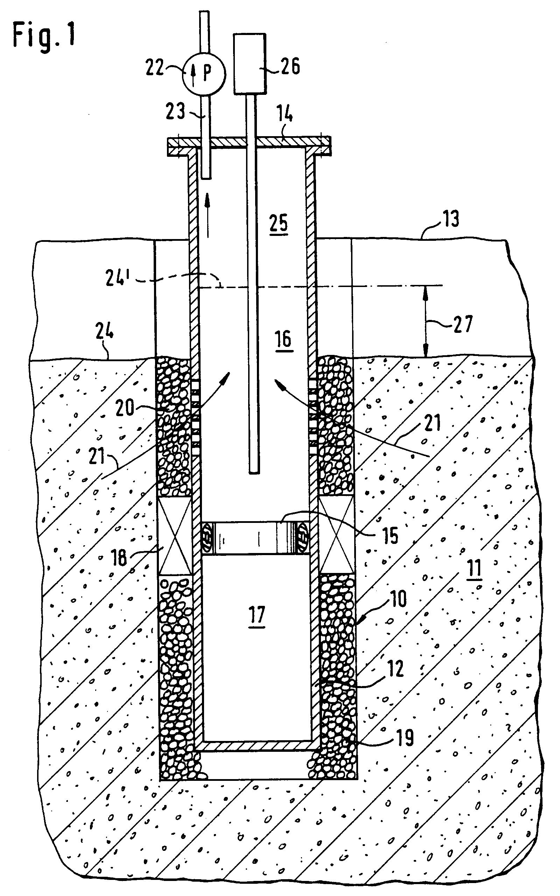

In Fig. 1 ist ein Brunnenschacht 10 dargestellt, der bis zu einem grundwasserdurchströmten Bodenbereich 11, dessen geohydraulische Durchlässigkeit bestimmt werden soll, getrieben ist. Der Schacht 10 ist mit einem Rohr 12 ausgekleidet, das oberhalb der Erdoberfläche 13 mit einem Deckel 14 verschlossen ist, und durch eine Trennwandung 15 in einen oberen Bereich 16 und einen unteren Bereich 17 aufgeteilt. In Höhe der Trennwandung 15 ist das Rohr 12 von einer Dichtungsmanschette 18 umgeben. In den übrigen Bereichen umgibt das Rohr 12 eine Filterkiesschüttung 19. Im oberen Schachtbereich 16 weist das Rohr 12 Wasserdurchgangsöffnungen 20 auf, durch die Grundwasser ins Schachtinnere strömen kann, was durch die beiden Pfeile 21 angedeutet ist. Außerhalb des Brunnenschachtes 10 ist eine Saugpumpe 22 angeordnet, die über ein Rohr 23 mit einem oberhalb des Grundwasserspiegels 24 liegenden Bereich 25 des Rohres 12 verbunden ist. Mittels der Saugpumpe 22 wird in diesem Bereich 25 ein Unterdruck erzeugt. Dadurch steigt der Grundwasserspiegel im Brunnenschacht 10 gegenüber dem Ruhepegel 24 auf ein Niveau 24' an. Dieser Anstieg des Grundwasserspiegels wird ebenso wie der im Bereich 25 herrschende Unterdruck von einer Meßsonde 26, die von außerhalb durch den Deckel 14 bis in den Schachtbereich 16 geführt ist, gemessen. Anstatt der Messung des Anstiegs des Grundwasserspiegels oder auch zusätzlich zu dieser kann auch eine Messung des Wiederabsinkens des Grundwasserspiegels pro Zeiteinheit vorgenommen werden. Aus diesen Meßwerten läßt sich dann die geohydraulische Durchlässigkeit des Erdreichs 11 berechnen, wobei mit der Vorrichtung nach Fig. 1 im wesentlichen die Durchlässigkeit des Bodens 11 in horizontaler Richtung im Bereich der Wasserdurchgangsöffnungen 20 im oberen Schachtbereich 16 ermittelt wird.

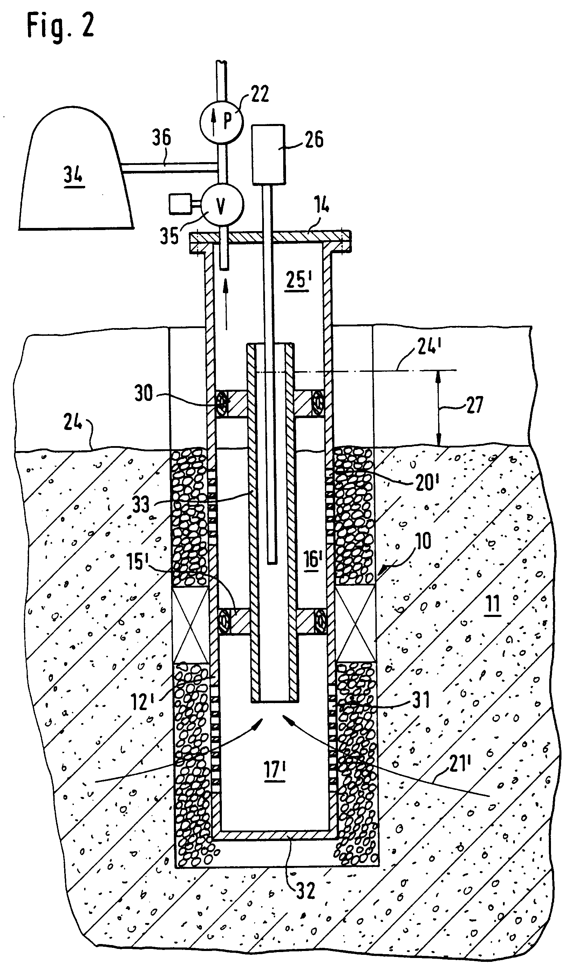

Fig. 2 zeigt eine Vorrichtung, mit der die horizontale Durchlässigkeit des Bodens 11 in zwei unterschiedlichen Tiefen gemessen werden kann. Auch hier ist der Schacht 10 von einem Rohr 12' ausgekleidet, das hier jedoch durch zwei Trennwandungen 15' und 30 in drei Bereiche 17', 16' und 25' aufgeteilt ist. Im Gegensatz zur Vorrichtung nach Fig. 1 weist hier das Rohr 12' Wasserdurchgangsöffnungen 20' und 31 in zwei verschiedenen Schachtbereichen, nämlich den Bereichen 16' und 17' auf. An seinem unteren Ende ist das Rohr von einer Bodenplatte 32 und an seinem oberen Ende, oberhalb der Erdoberfläche 13, von einem Deckel 14 verschlossen. Dadurch, daß das Rohr 12' in zwei unterschiedlichen Tiefen Wasserdurchgangsöffnungen 20' und 31 aufweist, kann die Durchlässigkeit des Bodens 11 in zwei unterschiedlichen Tiefen gemessen werden. In Fig. 2 ist die Messung der Durchlässigkeit in der dem unteren Schachtbereich 17' entsprechenden Tiefe des Bodenbereichs dargestellt. Zu diesem Zweck wird der obere Schachtbereich 16' durch ein Rohr 33, das durch die beiden Trennwandungen 30 und 15' vom Schachtbereich 25' oberhalb des Grundwasserspiegels 24 bis zum untersten Schachtbereich 17' geführt ist, überbrückt. Außerhalb des Schachtes 10 ist wieder eine Saugpumpe 22 zur Unterdruckerzeugung im Schacht 10 angeordnet. Mit der Saugpumpe 22 wird hierzu zunächst in einer Vakuumkammer 34 ein vorgebbarer Unterdruck erzeugt, bevor dieser durch Öffnen eines Ventils 35 an einer Verbindungsleitung 36 zwischen Vakuumkammer 34 und Brunnenschacht 10 auf letzteren übertragen wird. Der Unterdruck im Schacht 10 bewirkt wieder ein Ansteigen des Grundwasserspiegels vom Ruhepegel 24 auf einen erhöhten Pegel 24', wobei diese Anhöhung des Wasserspiegels ausschließlich durch in den Schachtbereich 17' zufließendes Grundwasser, was durch die Pfeile 21' angedeutet ist, erzeugt wird. Durch die Überbrückung des Bereiches 16' mit dem Rohr 33 macht sich der Unterdruck in diesem Schachtbereich nicht bemerkbar. Der Anstieg des Grundwasserspiegels auf das Niveau 24' ist somit nur im Rohr 33 feststellbar, wo die Änderung dieses Anstiegs 27 pro Zeiteinheit von einer Meßsonde 26 erfaßt wird. Aus diesen Meßwerten und dem bekannten Unterdruck im Schacht 10 läßt sich dann die geohydraulische Durchlässigkeit des Bodens 11 in der dem Schachtbereich 17' entsprechenden Tiefe in horizontaler Richtung berechnen. Wenn die horizontale geohydraulische Durchlässigkeit des Bodens 11 in einer Tiefe, die dem Schachtbereich 16' entspricht, gemessen werden soll, wird die Trennwandung 30 und das Durchgangsrohr 33 aus dem Brunnenschacht entfernt. Der im Schachtbereich 25' erzeugte Unterdruck wirkt dann nur auf den oberen Bereich 16' des Schachtes ein, so daß ausschließlich Wasser durch die Durchgangsöffnungen 20' im Rohr 12' ins Schachtinnere strömen kann. Die Meßanordnung entspricht dann derjenigen in Fig. 1.

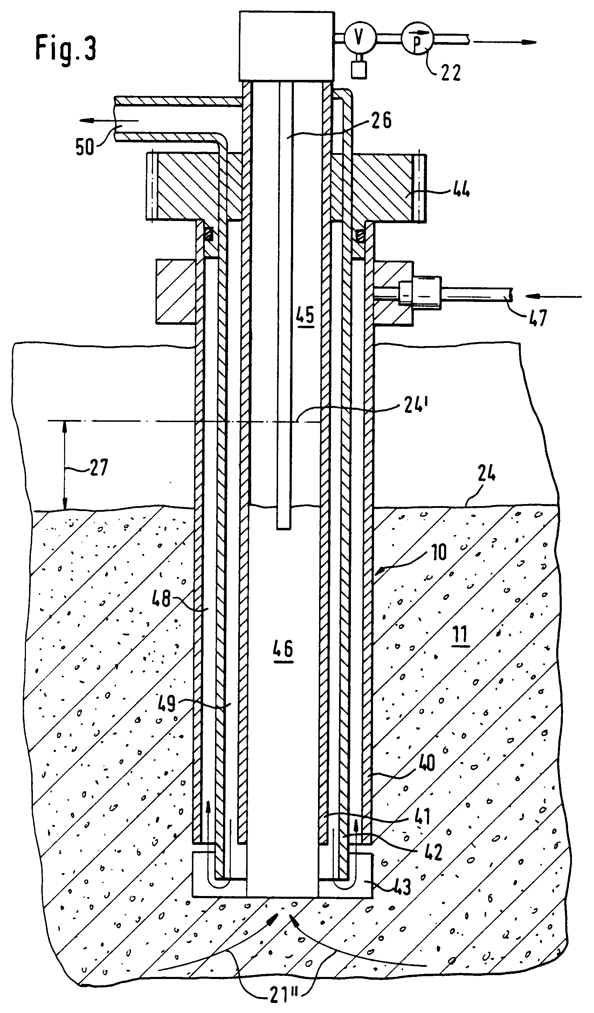

In Fig. 3 ist eine Vorrichtung zur Messung der vertikalen geohydraulischen Durchlässigkeit des Bodens 11 dargestellt. Der Brunnenschacht 10 wird hier durch drei konzentrische Rohre, einem äußeren Stützrohr 40, einem inneren Stützrohr 41 sowie einem mittleren Bohrrohr 42 gebildet.

Das Bohrrohr 42 ist an seinem unteren Ende mit einem Drehmeißel 43 versehen. An seinem oberen Ende ist ein Zahnkranz 44 angeordnet, über den das Rohr 42 angetrieben wird. Das Innere des Schachtes 10 ist nur in einem Bereich 45 oberhalb des Ruhegrundwasserspiegels 24 vom Erdreich befreit. Dies wird dadurch erreicht, daß beim Einbohren der Anordnung ins Erdreich 11 bei Erreichen des Wasserspiegels 24 das innere Stützrohr 41 zusammen mit dem Bohrkern aus Erdreich aus dem mittleren Rohr 42 herausgezogen wird. Anschließend wird das Rohr 41 wieder eingesetzt und die gesamte Anordnung bis zur gewünschten Tiefe durch Bohren mit dem mittleren Rohr 42 ins Erdreich 11 vorgetrieben. Im Bereich 45 des Schachtes 10 wird wieder mittels einer Saugpumpe 22 ein Unterdruck erzeugt. Dies bewirkt ein Ansteigen des Wasserspiegels auf ein Niveau 24' im Schachtinneren, wobei dieser Anstieg durch Zufließen von Grundwasser von unten in das innere Rohr 41 bewirkt wird. Das Wasser muß dabei den im unteren Schachtbereich 46 noch vorhandenen Bohrkern aus Erdreich durchströmen. Durch Messung des im Schachtbereich 45 erzeugten Unterdrucks sowie des Anstiegs 27 des Grundwasserspiegels 24 bzw. dessen Wiederabsinken pro Zeiteinheit mittels einer Meßsonde 26 kann damit die vertikale geohydraulische Durchlässigkeit des Bodens 11 gemessen werden. Zur Erleichterung des Bohrvorgangs des Bohrrohres 42 kann durch eine Öffnung 47 Spülwasser in die Rohrzwischenräume 48 und 49 gepreßt werden, mit dem Erdreich, das durch den Drehmeißel 43 gelockert worden ist, durch einen Ausgang 50 ausgespült werden kann.

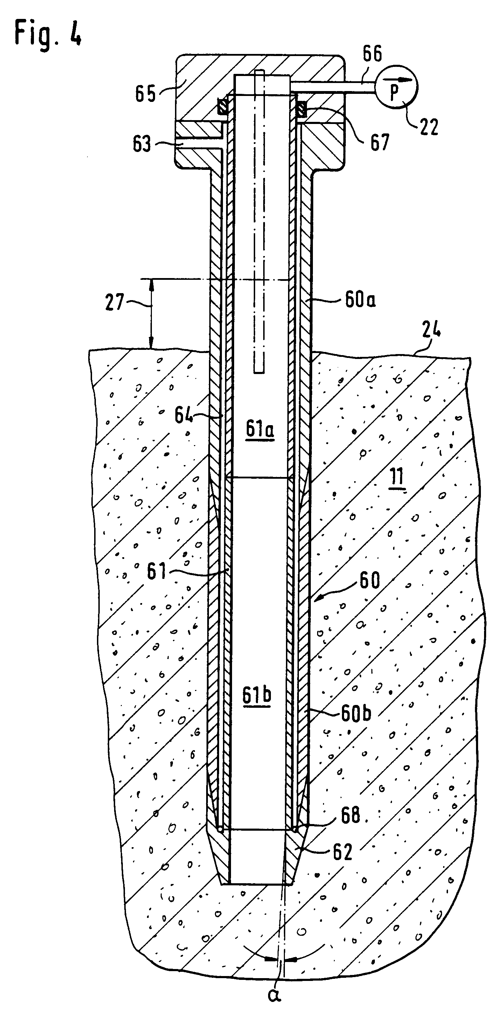

Fig. 4 zeigt eine zweite Ausführungsform einer Vorrichtung zur Messung der vertikalen Durchlässigkeit. In einem äußeren Pegelrohr 60 ist ein transparentes Innenrohr 61 entnehmbar angeordnet. Das Pegelrohr 60 besteht aus miteinander verschraubten Rohrelementen 60a, 60b und weist an seinem unteren Ende ein gehärtetes und zugespitztes Endstück 62 zur Erleichterung des Eintreibens des Rohres 60 in den Boden 11 auf. Das Pegelrohr 60 kann jedoch auch ein Außengewinde aufweisen, mit dem es ins Erdreich 11 eindrehbar ist. Das Endstück 62 läuft zur Vermeidung eines zu starken radialen Drucks des Kernes auf das Innenrohr um einen kleinen Winkel α konisch nach innen zu und kann ebenfalls mit einem Außengewinde versehen sein, um das Eindrehen der Anordnung in den Boden zu erleichtern. Das Innenrohr 61, das unten auf dem Endstück 62 des Pegelrohres 60 aufsitzt und über Dichtringe 67 und 68 gegen das Pegelrohr 60 abgedichtet ist, kann aus stumpf miteinander verschweißten Rohrelementen 61a, 61b zusammengesetzt werden. Durch die transparente Ausführung des Innenrohres 61 läßt sich der mit ihm entnommene unzerstörte Bohrkern rasch optisch untersuchen und erlaubt somit erste Rückschlüsse auf die Zusammensetzung des Erdreichs 11. An seinem oberen Ende weist das Pegelrohr 60 eine Entlüftungsöffnung 63 für den zwischen den Rohren 60 und 61 liegenden Freiraum 64 zur Verminderung der Bildung von Unterdruck beim Herausziehen des Innenrohres 61 aus dem Pegelrohr 60 und einen Deckel 65, an dem eine Meßsonde 26 angeordnet ist, auf. Durch den Deckel 65 führt ein Rohr 66 zu einer Saugpumpe 22 zur Erzeugung von Unterdruck in der Vorrichtung.

## Patentansprüche

1. Verfahren zur Bestimmung der geohydraulischen Durchlässigkeit von grundwasserdurchströmten Bodenbereichen, insbesondere für die Dimensionierung eines einen Grundwasser-Kreislauf erzeugenden Brunnens, dadurch gekennzeichnet, daß in einem Brunnenschacht (10) im zu untersuchenden Bodenbereich (11) oberhalb des Grundwasserspiegels (24) ein Unterdruck einstellbarer Größe erzeugt wird und der dadurch bewirkte Anstieg (27) des Grundwasserspiegels im Brunnenschacht (10) pro Zeiteinheit sowie der dort herrschende Unterdruck und/oder das Absinken des Grundwasserspiegels (24) pro Zeiteinheit nach Beseitigung des Unterdrucks gemessen und aus diesen Meßwerten die geohydraulische Durchlässigkeit des untersuchten Bodenbereichs (11) berechnet wird.

2. Verfahren nach Anspruch 1, dadurch gekennzeichnet, daß nacheinander der Anstieg (27) und/oder das Absinken des Grundwasserspiegels (24) bei Zufluß von Grundwasser aus verschiedenen Richtungen in einen Brunnenschacht (10) pro Zeiteinheit gemessen und daraus die Anisotropie der geohydraulischen Durchlässigkeit des Bodenbereichs (11) berechnet wird.

3. Verfahren nach Anspruch 2, dadurch gekennzeichnet, daß nacheinander der Anstieg (27) und/oder das Absinken des Grundwasserspiegels (24) bei Zufluß von Grundwasser aus horizontaler Richtung und aus vertikaler Richtung in einem Brunnenschacht (10) gemessen wird.

4. Verfahren nach Anspruch 2 oder 3, dadurch gekennzeichnet, daß jeweils der Anstieg (27) und/oder das Absinken des Grundwasserspiegels (24) bei Zufluß von Grundwasser aus horizontaler Richtung in unterschiedlichen Tiefen gemessen wird.

5. Vorrichtung zur Durchführung des Verfahrens nach einem der Ansprüche 1 bis 4, mit einem Brunnenschacht (10) mit bereichsweise wasserdurchlässiger Schachtwandung, dadurch gekennzeichnet, daß der Brunnenschacht (10) oberhalb des Grundwasserspiegels (24) luft- und wasserundurchlässig abgedichtet ist, Einrichtungen (22, 34, 35, 36) zur Unterdruckerzeugung und eine Meßsonde (26) zur Messung der Veränderung des Grundwasserspiegels (24) pro Zeiteinheit und des im Schacht (10) herrschenden Unterdrucks aufweist.

6. Vorrichtung nach Anspruch 5, dadurch gekennzeichnet, daß die Einrichtungen zur Unterdruckerzeugung (22, 34 bis 36) aus einer Saugpumpe (22), einer Vakuumkammer (34) und einem Ventil (35) bestehen und außerhalb des Brunnenschachts (10) angeordnet und mit diesen über ein Rohr (23, 36) verbunden sind.

7. Vorrichtung nach Anspruch 5 oder 6, dadurch gekennzeichnet, daß die Meßsonde (26) mit einer außerhalb des Brunnenschachts (10) angeordneten Auswerteeinrichtung verbunden ist.

8. Vorrichtung nach einem der Ansprüche 5 bis 7, dadurch gekennzeichnet, daß der Brunnenschacht zur Messung der horizontalen geohydraulischen Durchlässigkeit des zu untersuchenden Bodenbereichs (11) einen oder mehrere horizontale Bereiche (16, 17, 16', 17') mit wasserdurchlässiger Schachtwandung (20, 31), die gegeneinander abgedichtet sind, aufweist.

9. Vorrichtung nach Anspruch 8, dadurch gekennzeichnet, daß bei mehreren horizontalen Schachtbereichen (16', 17') der oder die oberen Schachtbereiche (16') durch ein durch die Abdichtungen (15', 30) zwischen den Bereichen (16', 17', 25) bis oberhalb des Grundwasserspiegels (24) im Brunnenschacht (10) geführtes Rohr (33) überbrückbar sind.

10. Vorrichtung nach einem der Ansprüche 5 bis 7, dadurch gekennzeichnet, daß der Brunnenschacht (10) zur Messung der vertikalen geohydraulischen Durchlässigkeit des zu untersuchenden Bodenbereichs (11) von mindestens einem ins Erdreich getriebenen, unten offenen Rohr (40, 41, 42) gebildet ist, dessen Inneres nur im oberhalb des Grundwasserspiegels (24) liegenden Bereich (45) von Erdreich befreit ist.

11. Vorrichtung nach Anspruch 10, dadurch gekennzeichnet, daß das Rohr oder die Rohre (40, 41, 42) einen kleinen Durchmesser aufweisen und in den zu untersuchenden Bodenbereich (11) einschlagbar oder eindrehbar sind.

12. Vorrichtung nach Anspruch 10, dadurch gekennzeichnet, daß der Brunnenschacht (10) zur Messung der vertikalen geohydraulischen Durchlässigkeit des zu untersuchenden Bodenbereichs (11) von drei konzentrischen Rohren (40, 41, 42) gebildet ist, wobei das mittlere Rohr (42) an seinem unteren Ende einen Drehmeißel (43) aufweist und an seinem oberen Ende mit einem Drehantrieb verbunden ist und das innere Rohr (41) zusammen mit dem Bohrkern aus dem mittleren Rohr (42) herausnehmbar ist.

13. Vorrichtung nach Anspruch 12, dadurch gekennzeichnet, daß sie eine Einrichtung zum Einpressen von Spülwasser in den Raum (48, 48) zwischen den Rohren (40, 41, 42) zum Ausschwemmen von beim Bohrvorgang gelöstem Erdreich aufweist.

14. Vorrichtung nach Anspruch 10, dadurch gekennzeichnet, daß der Brunnenschacht (10) zur Messung der vertikalen geohydraulischen Durchlässigkeit des zu untersuchenden Bodenbereichs (11) von zwei konzentrischen Rohren (60, 61) gebildet ist, wobei das Innenrohr aus einem transparenten, flexiblen Material gefertigt ist und mit seinem unteren Ende auf einem gehärteten, zugespitzten Endstück (62) des Außenrohres (60) aufsitzt.

15. Vorrichtung nach Anspruch 14, dadurch gekennzeichnet, daß zwischen dem Innenrohr (61) und dem Außenrohr (60) ein entlüftbarer Freiraum (64) angeordnet ist.

16. Vorrichtung nach Anspruch 14 oder 15, dadurch gekennzeichnet, daß das Innenrohr aus stumpfgeschweißten Rohrstücken (61a, 61b) zusammengesetzt ist und das Außenrohr (60) ein Außengewinde zum Einbohren ins Erdreich (11) aufweist.
